# EUROPEAN PATENT APPLICATION

(11) **EP 4 198 048 A1**
(43) Date of publication of application: **21.06.2023**
(21) Application number: 21215277.1
(22) Date of filing: 16.12.2021
(51) Int. Cl.: C07K 14/47

(54) **CALPAIN-3 GENE TRANSFER INCREASE USING MODIFIED ITR SEQUENCES**

(71) Applicant: GENETHON, 91000 Evry-Courcouronnes (FR); Institut National de la Santé et de la Recherche Médicale, 75013 Paris (FR); UNIVERSITE D'EVRY-VAL-D'ESSONNE, 91000 Evry-Courcouronnes (FR)
(72) Inventor: RICHARD, Isabelle, 91100 CORBEIL-ESSONNES (FR); DORANGE, Fabien, 28630 NOGENT-LE-PHAYE (FR)
(74) Representative: Axe PI

(57) **Abstract**

The present invention relates to a gene expression construct comprising an expression cassette containing a transgene encoding a calpain-3 protein and modified ITR sequences, said transgene encoding the calpain-3 protein consisting of the sequence of SEQ ID NO: 4 or a functional variant thereof having a polynucleotide sequence that is at least 80% identical to SEQ ID NO: 4; said modified ITR sequences consisting of - a modified 5'-ITR sequence of SEQ ID NO: 1; and - a modified 3'-ITR sequence of SEQ ID NO: 2 or SEQ ID NO: 3. The invention also relates to a recombinant vector comprising the gene expression construct of the invention, said gene expression construct or recombinant vector can be included in a gene therapy and used as a medicament, in particular for the treatment of limb girdle muscular dystrophy by calpainopathy.

## Description

### FIELD OF THE INVENTION

The present invention relates to a gene expression construct containing modified ITR sequences and an expression cassette comprising a transgene encoding a calpain-3 protein. The invention also relates to a recombinant vector comprising the gene expression construct. This gene expression construct or recombinant vector can be used in a gene therapy, in particular for the treatment of limb girdle muscular dystrophy.

### BACKGROUND

Muscular dystrophies (MDs) are a group of genetic diseases. The group is characterized by progressive weakness and degeneration of the skeletal muscles that control movement. Some forms of MDs develop in infancy or childhood, while others may not appear until middle age or later. The disorders differ in terms of the distribution and extent of muscle weakness (some forms of MDs also affect cardiac muscle), the age of onset, the rate of progression, and the pattern of inheritance. One group of MDs is the limb girdle muscular dystrophies (LGMD).

LGMDs are rare conditions, and they present differently in different people with respect to age of onset, areas of muscle weakness, heart and respiratory involvement, rate of progression and severity. LGMDs can begin in childhood, adolescence, young adulthood or even later. Both genders are affected equally. LGMDs cause weakness in the shoulder and pelvic girdle, with nearby muscles in the upper legs and arms sometimes also weakening with time. Weakness of the legs often appears before that of the arms. Facial muscles are usually unaffected. As the condition progresses, people can have problems with walking and may need to use a wheelchair over time. The involvement of shoulder and arm muscles can lead to difficulty in raising arms over head and in lifting objects. Physical therapy is often recommended to retain muscle strength and mobility for as long as possible. Stretching, mechanical aids, or surgery may also aid in that goal. In some types of LGMD, the heart and breathing muscles may be involved. As muscles deteriorate, a ventilator may be required to help with breathing. Cardiac surveillance is recommended, and those who develop heart problems should consult a cardiologist for appropriate treatment.

LGMD is a heterogeneous group of genetic forms of muscular dystrophy with many subtypes that are each caused by a unique mutation generating compilation of symptoms.

There are several forms of LGMD, and the forms are classified by their associated genetic defects.

Calpain-3 is a calcium-dependent non lysosomal protease encoded by CAPN3 gene and is involved primarily in regulating sarcomere formation and remodeling. Additionally, calpain-3 serves structural functions as a component of the skeletal muscle triad and the dysferlin complex. Calpain-3 has been found in many subcellular locations, the majority of it is anchored to titin, a giant sarcomeric protein that acts as a molecular ruler and a template for sarcomere assembly during muscle cell development.

A mutation in the CAPN3 gene may cause limb girdle muscular dystrophy type R1 formerly 2A, also referred to as calpainopathy.

It is characterized by progressive weakness of the trunc and upper thigh muscles, usually beginning between 8 and 15 years old, with a loss of ambulation occurring 10 to 20 years later.

Clinically, calf hypertrophy is rarely observed, but Achille's tendon contractures are common. Progression of calpainopathy is variable. Most patients may have normal mobility in childhood with a very slowly progressive course of disease. Respiratory, but not cardiac, complications have been reported.

Gene therapy (also called gene transfer) is a medical field which focuses on the utilization of the therapeutic delivery of nucleic acids into a patient's cells as a drug to treat diseases. It is thought to be able to cure many genetic disorders or treat them over time.

The concept of gene therapy is to fix a genetic problem at its source. If, for instance, in an (usually recessively) inherited disease a mutation in a certain gene results in the production of a dysfunctional protein, gene therapy could be used to deliver a copy of this gene that does not contain the deleterious mutation (named transgene), and thereby produces a functional protein. This strategy is referred to as gene replacement therapy.

Most of these approaches utilize adeno-associated viruses (AAVs) and lentiviruses for performing nucleic acid insertions, in *vivo* and ex *vivo,* respectively. In more than 75% of gene therapy clinical trials, a viral vector is the vehicle.

Adeno-associated virus (AAV) is the most common and powerful vector, derived from adeno-associated human parvovirus. This small virus has attracted the attention of the gene therapy field because of its low risk (not pathogenic for human and no integrative genome), the remarkable ability of recombinant AAV (rAAV) vectors to transduce with high efficiency dividing and non-dividing cells in a large variety of tissues, long-term transgene expression after injection and specific tissue tropism.

In practice, the nucleic acid sequence of interest, usually placed under the control of regulatory sequences allowing its expression, replaces the viral genome between the ITR sequences.

Adeno-associated virus (AAV) is a small non-enveloped DNA virus composed by an icosahedral capsid that contains a 4.7 kb linear single-stranded genome. AAV genome codes for non-structural proteins (Rep78, 68, 52 and 40), capsid proteins (VP1, VP2, VP3) and the assembly-activating protein (AAP).

AAV genome is structurally characterized by about 145-bp Inverted Terminal Repeats (ITRs) that flank two open reading frames (ORFs). The first 125 nucleotides of the ITR constitute a palindrome, which folds upon itself to maximize base pairing and forms a T-shaped hairpin structure. The other 20 bases, called the D sequence, remain unpaired. The ITRs are important cis-active sequences in the biology of AAV. A key role of the ITRs is in AAV DNA replication. In the current model of AAV replication, the ITR is the origin of replication and serves as a primer for second-strand synthesis by DNA polymerase. The double-stranded DNA formed during the synthesis, called replicating-form monomer, is used for a second round of self-priming replication and forms a replicating-form dimer. These double-stranded DNA intermediates (replicating-form monomer and replicating-form dimer) are processed via a strand displacement mechanism, resulting in single-stranded DNA used for packaging and double-stranded DNA used for transcription. In addition to their role in AAV replication, the ITR is also essential for encapsidation of the viral genome, transcription, negative regulation under nonpermissive conditions, and site-specific integration.

In practice, the nucleic acid sequence of interest, usually placed under the control of regulatory sequences allowing its expression, replaces the viral genome between the ITR sequences.

Patent application WO2020/006458 discloses products and methods for treating limb girdle muscular dystrophy 2A with recombinant adeno-associated viruses delivering a protein with calpain 3 activity.

However, the efficiency of gene therapy is highly impacted by the quality of the recombinant AAV vector. Up to now, no more than one third of the industrially produced AAV vectors have integrated a full expression construct. It means that the majority of the AAV vectors injected to patient have no or truncated gene expression constructs. Such AAV vectors will thus be inefficient. Indeed, even if a truncated gene expression construct is delivered into the host cells, it will not be expressed.

Hence, there remains a need for gene therapy treatment with more efficient expression of the transgene of interest by transduced cells.

### PROBLEM TO BE SOLVED

The technical problem underlying the present invention is thus the provision of improved means and methods to generate higher quality recombinant AAV vectors, to increase in *vivo* gene transfer and expression of the calpain-3 protein.

So far, there is no satisfying treatment for calpainopathy.

The technical problem is solved by the embodiments provided herein below and as characterized in the appended claims.

### SUMMARY OF THE INVENTION

In a first aspect, the invention relates to a gene expression construct comprising:
- an expression cassette containing a transgene encoding a calpain-3 protein;
- a modified 5'-ITR sequence of SEQ ID NO: 1; and
- a modified 3'-ITR sequence of SEQ ID NO: 2 or SEQ ID NO: 3.

In another aspect, the invention relates to a recombinant AAV vector comprising the gene expression construct according to the invention.

In a further aspect, the invention relates to the gene expression construct according to the invention, or the recombinant AAV vector according to the invention, for use as a medicament.

The Applicant has been able to demonstrate that the use of an ITR sequence variant different from the wild-type ITR sequences (ITR WT) shows a surprisingly increase in the number of AAV vectors containing a complete gene expression construct.

The Applicant has also been able to observe an increase in the efficiency of gene transfer and expression of a calpain-3 protein.

A higher number of AAV capsids containing a complete gene expression construct present the advantage to decrease the quantity of AAV to administer to the patient and thus to minimize the unwanted activation of the immune system of the host.

A higher number of AAV capsids containing a complete gene expression construct also present the advantage to reduce the production costs of such AAV capsids clinical batches.

Further aspects and advantages of the present invention are described in the following description (with reference to Figures x to x), which should be regarded as illustrative and not limiting the scope of the present application.

### BRIEF DESCRIPTION OF THE DRAWINGS

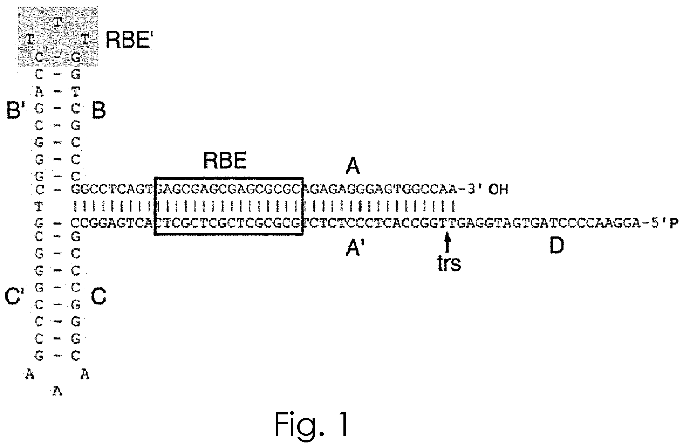

Figure 1. shows an ITR structure which comprises two palindromes (BB' and CC') integrated into a larger palindrome (AA') and a D sequence present only once at each end of the genome.

### DETAILED DESCRIPTION

In this description, unless it is specified otherwise, it is understood that, when an interval is given, it includes the upper and lower bounds of said interval.

The present invention provides materials and methods useful in therapy, more particularly for the treatment of Limb girdle muscular dystrophy. More specifically, the present invention provides combinations of regulatory elements useful for the improved expression of calpain-3 protein, such as modified ITR sequences.

The advantages of the invention are more particularly shown with respect to the treatment of Limb girdle muscular dystrophy.

The invention relates, in a first aspect, to a gene expression construct, comprising:
- an expression cassette containing a transgene encoding a Limb girdle protein;
- a modified 5'-ITR sequence of SEQ ID NO: 1; and
- a modified 3'-ITR sequence of SEQ ID NO: 2 or SEQ ID NO: 3.

The "gene expression construct" as used herein refers to a construction comprising an expression cassette, a modified 5'-ITR sequence and a modified 3'-ITR sequence, both ITR sequences surrounding the expression cassette.

The "expression cassette" as used herein includes a transgene of interest encoding a polypeptide to be expressed and polynucleotide sequences controlling its expression such as a promoter and a polyadenylation signal. Such cassette may also comprise an intron which is defined as a non-coding nucleic acid sequence which can modify gene expression (e.g. enhancing expression properties). Such intron may be for example an hemoglobin (HBB2) intron or a human β globin gene intron.

The term "polynucleotide(s)" as used herein generally refers to a plurality, two or more, of "nucleotide(s)", i.e. any polyribonucleotide or polydeoxyribonucleotide, which may be unmodified RNA or DNA or modified RNA or DNA. Polynucleotide(s) include, without limitation, single- and double-stranded DNA, DNA that is a mixture of single- and double-stranded regions or single- and triple stranded regions, single- and double-stranded RNA, and RNA that is a mixture of single and double-stranded regions, hybrid molecules comprising DNA and RNA that can be single-stranded or, more typically, double-stranded, or triple-stranded regions, or a mixture of single- and double-stranded regions. As used herein, the term "polynucleotide(s)" also includes DNAs or RNAs as described above that contain one or more modified bases. Thus, DNAs or RNAs with backbones modified for stability or for other reasons are "polynucleotide(s)" as the term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term "polynucleotide(s)" as used herein embraces such chemically, enzymatically, or metabolically modified forms of polynucleotides, as well as the chemical forms of DNA and RNA characteristic of viruses and cells, including, for example, simple and complex cells. "Polynucleotide(s)" also embraces short polynucleotides often referred to as oligonucleotide(s).

The term "polypeptide(s)" as used herein refers to any peptide or protein comprising two or more amino acids joined to each other by peptide bonds or modified peptide bonds. "Polypeptide(s)" refers to both short chains, commonly referred to as peptides, oligopeptides, and oligomers and to longer chains generally referred to as proteins. Polypeptides can contain amino acids other than the gene encoded amino acids. "Polypeptide(s)" include those modified either by natural processes, such as processing and other post-translational modifications.

The term "transgene" refers to a gene whose nucleic acid sequence is non-naturally occurring in the genome of a subject undergoing LGMD R1. The transgene used herein is the wild type human CAPN3 gene.

In another particular embodiment, the transgene sequence encoding the calpain-3 protein, in particular the human calpain-3 protein, is optimized.

Sequence optimization may include a number of changes in a nucleic acid sequence, including codon optimization, increase of GC content, decrease of the number of CpG islands, decrease of the number of alternative open reading frames (ARFs) and/or decrease of the number of splice donor and splice acceptor sites.

Because of the degeneracy of the genetic code, different nucleic acid molecules may encode the same protein. It is also well known that the genetic codes of different organisms are often biased towards using one of the several codons that encode the same amino acid over the others. Through codon optimization, changes are introduced in a polynucleotide sequence that take advantage of the codon bias existing in a given cellular context so that the resulting codon optimized polynucleotide sequence is more likely to be expressed in such given cellular context at a relatively high level compared to the non-codon optimized sequence.

In a preferred embodiment of the invention, such sequence optimized polynucleotide sequence encoding a calpain-3 protein, is codon-optimized to improve its expression in human cells compared to non-codon optimized polynucleotide sequences coding for the same protein (e.g. a calpain-3 protein), for example by taking advantage of the human specific codon usage bias.

In a particular embodiment, the optimized coding sequence (e.g. a calpain-3 coding sequence) is codon optimized, and/or has an increased GC content and/or has a decreased number of alternative open reading frames, and/or has a decreased number of splice donor and/or splice acceptor sites, as compared to the wild-type coding sequence.

In a particular embodiment, the nucleic acid sequence of the transgene encoding the calpain-3 protein is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 4, more preferably consists of the sequence of SEQ ID NO: 4.

"Identity", as used herein, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in the following references (Computational Molecular Biology, Lesk A.M., ed., Oxford University Press, New York, 1988; Biocomputing: Informatics and genome Projects, Smith D.W., ed., Academic Press, New York. 1993; Computer Analysis of sequence Data, Part I, Griffin A.M., and Griffin H.G., eds., Humana Press. New jersey, 1994; sequence Analysis in Molecular Biology, von Heinje G., Academic Press, 1987; and sequence Analysis Primer, Gribskov M. and Devereux J., eds., M Stockton Press, New York, 1991; and Carillo H., and Lipman D., SIAM J. Applied Math., 48:1073 (1998)). Methods to determine identity are designed to give the largest match between the sequences tested. Moreover, methods to determine identity are codified in publicly available computer programs. Computer program methods to determine identity between two sequences include, but are not limited to, the GCG program package (Devereux J. et al., Nucleic Acids Research 12(1): 387 (1984)), BLASTP, BLASTN, and FASTA (Altschul S.F. et al., J. Molec. Biol. 215: 403-410 (1990)). The BLAST X program is publicly available from NCBI and other sources (BLAST Manual, Altschul S. et al., NCBI NLM NUH Bethesda, MD 20894; Altschul S. et al., J. Mol Biol. 215: 403-410 (1990)).

The term "variant(s)" as used herein, is a polynucleotide or polypeptide that differs from a reference polynucleotide or polypeptide respectively but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and/or truncations in the polypeptide encoded by the reference sequence, as discussed below. A typical variant of a polypeptide differs in amino acid sequence from another reference polypeptide.

Generally, differences are limited so that the sequences of the reference polypeptide and the variant are closely similar overall and, in many regions, identical. A variant and reference polypeptide may differ in amino acid sequence by one or more substitutions, additions, and/or deletions in any combination.

The present invention also includes variants of each of the polypeptides of the invention, that is polypeptides that vary from the references by conservative amino acid substitutions, whereby a residue is substituted by another with like characteristics. Typical conservative amino acid substitutions are among Ala, Val, Leu and Ile; among Ser and Thr; among the acidic residues, Asp and Glu; among Asn and Gln; and among the basic residues Lys and Arg; or aromatic residues Phe and Tyr. Such conservative mutations include mutations that switch one amino acid for another within one of the following groups:
1. Small aliphatic, nonpolar or slightly polar residues: Ala, Ser, Thr, Pro and Gly;
2. Polar, negatively charged residues and their amides: Asp, Asn, Glu and Gln;
3. Polar, positively charged residues: His, Arg and Lys;
4. Large aliphatic, nonpolar residues: Met, Leu, Ile, Val and Cys; and
5. Aromatic residues: Phe, Tyr and Trp.

Particularly preferred are variants in which several, e.g., 5-10, 1-5, 1-3, 1-2 or 1 amino acids are substituted, deleted, or added in any combination. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to a person skilled in the art.

As mentioned above, in addition to the GC content and/or number of ARFs, sequence optimization may also comprise a decrease in the number of CpG islands in the sequence and/or a decrease in the number of splice donor and acceptor sites.

Of course, as is well known to those skilled in the art, sequence optimization is a balance between all these parameters, meaning that a sequence may be considered optimized if at least one of the above parameters is improved while one or more of the other parameters is not, as long as the optimized sequence leads to an improvement of the transgene, such as an improved expression and/or a decreased immune response to the transgene in *vivo.*

The gene expression construct according to the invention also comprises modified ITR sequences.

The ITR sequences (ITR for Inverted Terminal Repeats) are palindromic sequences of 145 nucleotides. They are present at the 5' and 3' ends of the wild-type AAV DNA strand and are necessary for viral genome replication and packaging.

The ITR sequences have distinct parts as illustrated in Figure 1. Each ITR sequence comprises two palindromes (BB' and CC') integrated into a larger palindrome (AA') on either side of the genome of the AAV and a D sequence present only once at each end of the genome. The ITR sequences include other additional elements such as a Rep binding element (RBE) where the AAV proteins Rep78 and Rep68 are bounded, an additional RBE (RBE') which allows to stabilize the association between Rep78 and Rep 68 proteins and the ITR and a terminal resolution site (trs).

In a particular embodiment, the nucleic acid sequence encoding a modified 5'-ITR sequence is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 1, more preferably consists of the sequence of SEQ ID NO: 1.

In another particular embodiment, the nucleic acid sequence encoding a modified 3'-ITR is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 2 or SEQ ID NO: 3, more preferably consists of the sequence of SEQ ID NO: 2 or SEQ ID NO: 3.

In another embodiment, the expression cassette according to the invention also comprises a promoter, and optionally a polyadenylation signal.

In another preferred embodiment, the expression cassette according to the invention also comprises a promoter, a polyadenylation signal and preferably an intron with, for example, enhancing expression properties.

In a particular embodiment, the promoter comprised in the expression cassette according to the invention may be a desmin promoter, a MCK promoter, a SPc5-12 promoter or an alpha-SMA promoter.

In a preferred embodiment, the promoter is a desmin promoter which is a functional variant of the sequence of SEQ ID NO: 5, having a polynucleotide sequence that is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 5, more preferably consists of SEQ ID NO: 5.

In the context of the present invention, a functional variant of the desmin promoter is a sequence deriving therefrom by one or more nucleotide modifications, such as nucleotide substitution, addition or deletion, that results in the same or substantially the same level of expression (e.g. ± 20%, such as ± 10%, ± 5% or ± 1 %) of the CAPN3 transgene operatively linked thereto.

In a particular embodiment, the polyadenylation signal in the expression cassette of the invention may be derived from a number of genes. Illustrative polyadenylation signals include, without limitation, the HBB2 polyadenylation signal, the bovine growth hormone polyadenylation signal and the SV40 polyadenylation signal.

In a preferred embodiment, the polyadenylation signal is a HBB2 polyadenylation signal which is a functional variant of the sequence of SEQ ID NO: 6, having a polynucleotide sequence that is at least 80% identical, preferably at least 81%, 82%, 83%, 84%, 85%, 86%, 87%, 88%, 89%, 90%, 91%, 92% 93%, 94%, 95%, 96% 97%, 98% or 99% identical to the sequence of SEQ ID NO: 6, more preferably consists of SEQ ID NO: 6.

In the context of the present invention, a functional variant of the HBB2 polyadenylation signal is a sequence deriving therefrom by one or more nucleotide modifications, such as nucleotide substitution, addition or deletion, that results in the same or substantially the same level of expression (e.g. ± 20%, such as ± 10%, ± 5% or ± 1 %) of the calpain-3 transgene operatively linked thereto.

A preferred aspect of the invention provides a gene expression construct comprising an expression cassette also comprising:
- a desmin promoter consisting of the sequence of SEQ ID NO: 5 or a functional variant thereof having a polynucleotide sequence that is at least 80% identical to SEQ ID NO: 5; and optionally
- an HBB2 polyadenylation signal consisting of the sequence of SEQ ID NO: 6 or a functional variant thereof having a polynucleotide sequence that is at least 80% identical to SEQ ID NO: 6.

In another particular preferred embodiment, the expression cassette of the gene expression construct comprises:
- a desmin promoter consisting of the sequence of SEQ ID NO: 5 or a functional variant having a polynucleotide sequence that is at least 80% identical to SEQ ID NO: 5;
- a transgene encoding the calpain-3 protein consisting of the sequence SEQ ID NO: 4 or a functional variant thereof having a polynucleotide sequence that is at least 80% identical to SEQ ID NO: 4; and
- an HBB2 polyadenylation signal consisting of the sequence of SEQ ID NO: 6 or a functional variant thereof having a polynucleotide sequence that is at least 80% identical to SEQ ID NO: 6.

The gene expression construct of the invention can be included in a recombinant vector.

A further aspect of the invention relates to a recombinant vector comprising a gene expression construct as described above.

A recombinant vector can be used in gene therapy.

As used herein, the term "gene therapy" refers to the transfer of genetic material (e.g., DNA or RNA) of interest into a host to treat or prevent a genetic or acquired disease or condition. The genetic material of interest encodes a product (e.g., a polypeptide or functional RNA) whose production is desired in *vivo.* For example, the genetic material of interest can encode a hormone, receptor, enzyme or polypeptide of therapeutic value. Alternatively, the genetic material of interest can encode a functional RNA of therapeutic value, such as an antisense RNA or a shRNA of therapeutic value.

In a particular embodiment, the recombinant vector is a viral vector.

In another particular embodiment, the recombinant vector of the invention is a recombinant viral AAV (rAAV) vector.

In the context of the present invention, the terms "adeno-associated virus" (AAV) and "recombinant adeno-associated virus" (rAAV) are used interchangeably herein and refer to an AAV whose genome was modified, as compared to a wild-type (wt) AAV genome, by replacement of a part of the wt genome with a transgene of interest.

Recombinant AAVs may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus. Desirable AAV elements for assembly into vectors include the cap proteins, including the vp1, vp2, vp3 and hypervariable regions, the rep proteins, including rep 78, rep 68, rep 52, and rep 40, and the sequences encoding these proteins. These elements may be readily used in a variety of vector systems and host cells.

In the present invention, the capsid of the AAV vector may be derived from a naturally or non-naturally occurring serotype.

In a particular embodiment, the serotype of the capsid of the AAV vector is selected from AAV natural serotypes.

Alternatively to using AAV natural serotypes, artificial AAV serotypes may be used in the context of the present invention, including, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. A capsid from an artificial AAV serotype may be, without limitation, a chimeric AAV capsid, a recombinant AAV capsid, or a "humanized" AAV capsid.

In a particular embodiment, the AAV vector has a naturally occurring capsid, such as an AAV1, AAV2, AAV3, AAV4, AAV5, AAV6, AAV7, AAV8, AAV9, AAV9-rh74, AAV-cy10, AAVrh10, AAV11 and AAV12 capsid.

In a particular embodiment, the capsid of the AAV vector is selected from an AAV9 or AAV9-rh74 capsid.

In a particular embodiment, the AAV vector is an AAV vector with high tropism to muscle cells and/or cardiac cells. In a variant of this embodiment, the AAV vector has an AAV8, AAV9, AAV9-rh74 capsid.

In particular embodiments of the invention, a rAAV vector may comprise an AAV9 or AAV9-rh74 capsid. Such vector is herein termed "AAV9 vector" or "AAV9-rh74 vector", respectively, independently of the serotype the genome contained in the rAAV vector is derived from. Accordingly, an AAV9 vector may be a vector comprising an AAV9 capsid and an AAV9 derived genome or a pseudo-typed vector comprising an AAV9 capsid and a genome derived from a serotype different from the AAV9 serotype. Likewise, an AAV9-rh74 vector may be a vector comprising an AAV9-rh74 capsid and an AAV9-rh74 derived genome or a pseudo-typed vector comprising an AAV9-rh74 capsid, and a genome derived from a serotype different from the AAV9-rh74 serotype.

The genome present within the rAAV vector of the present invention may be single- stranded or self-complementary. In the context of the present invention a "single stranded genome" is a genome that is not self-complementary, i.e. the coding region contained therein has not been designed as disclosed in McCarty et al., 2001 and 2003 (Op. cif) to form an intra molecular double-stranded DNA template. On the contrary, a "self-complementary AAV genome" has been designed as disclosed in McCarty et al., 2001 and 2003 (Op. cif) to form an intra-molecular double-stranded DNA template.

In another particular embodiment, the pseudo-typed rAAV vector includes a gene expression construct, in particular a single-stranded genome, comprising:
- an expression cassette containing a transgene encoding the calpain-3 protein consisting of the sequence of SEQ ID NO: 4 or a functional variant thereof having a polynucleotide sequence that is at least 80% identical to SEQ ID NO: 4;
- a modified 5'-ITR sequence of SEQ ID NO: 1; and
- a modified 3'-ITR sequence of SEQ ID NO: 2 or SEQ ID NO: 3
   and a capsid selected in the group consisting of an AAV9 and AAV9-rh74 capsid.

In a preferred embodiment, in particular in a variant wherein the gene expression construct is a single stranded AAV genome (which is not self-complementary as explained above), the gene expression construct has a size comprised between 2100 and 5000 nucleotides, in particular between 2700 and 4900 nucleotides, more particularly between 3200 and 4700 nucleotides. In a particular embodiment, the size of the gene expression construct is of about 3200 nucleotides, about 3300 nucleotides, about 3400 nucleotides, about 3500 nucleotides, about 3600 nucleotides, about 3700 nucleotides, about 3800 nucleotides, about 3900 nucleotides, about 4000 nucleotides, about 4100 nucleotides, or about 4200 nucleotides.

According to the present invention, the term "about", when referring to a numerical value, means plus or minus 5% of this numerical value.

In another aspect, the invention provides DNA plasmids comprising rAAV genomes of the invention. Production of rAAV requires that the following components are present within a single cell (denoted herein as a packaging cell): a rAAV genome, AAV rep and cap genes separate from (i.e., not in) the rAAV genome, and helper virus functions. Production of pseudotyped rAAV is disclosed in, for example, WO 01/83692. Production may implement transfection a cell with two, three or more plasmids. For example, three plasmids may be used, including: (i) a plasmid carrying a Rep/Cap cassette, (ii) a plasmid carrying the rAAV genome (i.e. a transgene flanked with AAV ITRs) and (iii) a plasmid carrying helper virus functions (such as adenovirus helper functions). In another embodiment, a two-plasmid system may be used, comprising (i) a plasmid comprising Rep and Cap genes, and helper virus functions, and (ii) a plasmid comprising the rAAV genome.

In a further aspect, the invention relates to a plasmid comprising the isolated nucleic acid construct of the invention. This plasmid may be introduced in a cell for producing a rAAV vector according to the invention by providing the rAAV genome to said cell.

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are incorporated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells. General principles of rAAV production are reviewed in, for example, Carter, 1992, Current Opinions in Biotechnology, 1533-539; and Muzyczka, 1992, Curr. Topics in Microbial, and Immunol., 158:97-129). Various approaches are described in Ratschin et al., Mol. Cell. Biol. 4:2072 (1984); Hermonat et al., Proc. Natl. Acad. Sci. USA, 81 :6466 (1984); Tratschin et al., Mol. Cell. Biol. 5:3251 (1985); McLaughlin et al., J. Virol., 62: 1963 (1988); and Lebkowski et al., 1988 Mol. Cell. Biol., 7:349 (1988); Samulski et al. (1989, J. Virol., 63:3822-3828); U.S. Patent No. 5,173,414; WO95/13365 and corresponding U.S. Patent No. 5,658,776; WO95/13392; WO96/17947; PCT/US98/18600; WO97/09441 (PCT/US96/14423); WO97/08298 (PCT/US96/13872); WO97/21825 (PCT/US96/20777); WO97/06243 (PCT/FR96/01064); WO99/11764; Perrin et al. (1995) Vaccine 13: 1244- 1250; Paul et al. (1993) Human Gene Therapy 4:609-615; Clark et al. (1996) Gene Therapy 3: 1 124-1 132; U.S. Patent. No. 5,786,211; U.S. Patent No. 5,871,982; and U.S. Patent. No. 6,258,595. The invention thus also provides packaging cells that produce infectious rAAV. In one embodiment packaging cells may be stably transformed cancer cells such as HeLa cells, HEK293 cells, HEK293T, HEK293vc and PerC.6 cells (a cognate 293 line). In another embodiment, packaging cells are cells that are not transformed cancer cells such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells).

The rAAV may be purified by methods standard in the art such as by column chromatography or cesium chloride gradients. Methods for purifying rAAV vectors from helper virus are known in the art and include methods disclosed in, for example, Clark et ah, Hum. Gene Ther., 10(6): 1031-1039 (1999); Schenpp and Clark, Methods Mol. Med., 69: 427-443 (2002); U.S. Patent No. 6,566, 118 and WO 98/09657.

In another aspect, the invention provides compositions comprising a rAAV disclosed in the present application. Compositions of the invention comprise rAAV in a pharmaceutically acceptable carrier.

The compositions may also comprise other ingredients such as diluents and adjuvants. Acceptable carriers, diluents and adjuvants are nontoxic to recipients and are preferably inert at the dosages and concentrations employed, and include buffers such as phosphate, citrate, or other organic acids; antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics or polyethylene glycol (PEG).

Thanks to the present invention, the transgene encoding calpain-3 protein may be expressed efficiently in a tissue of interest for the treatment of LGMD, preferably limb girdle muscular dystrophy by calpainopathy (LGMD R1).

According to the invention, in another aspect of the invention, the gene expression construct comprises:
- an expression cassette containing a transgene encoding a calpain-3 protein;
- a modified 5'-ITR sequence of SEQ ID NO: 1; and
- a modified 3'-ITR sequence of SEQ ID NO: 2 or SEQ ID NO: 3;
   for use as a medicament.

Another aspect of the invention relates to the recombinant vector of the invention comprising the gene expression construct according to the invention for use as a medicament.

Alternatively, the invention also relates to the use of the gene expression construct or the recombinant vector of the invention described above, as a medicament.

A further aspect of the invention provides a pharmaceutical composition comprising a recombinant vector comprising the gene expression construct for use as a medicament according to preceding aspects of the invention such as previously mentioned, and a pharmaceutically acceptable medium.

A pharmaceutically acceptable medium includes any, and all solvents, dispersion media, coatings, antibacterial and antifungal agents, isotonic and absorption delaying agents and the like suitable for administration to a mammalian host. The use of such media and agents for pharmaceutical active substances is well known in the art. Supplementary active ingredients can also be incorporated into the medicament of the present invention.

The gene expression construct or recombinant vector comprising said gene expression construct according to the invention are preferably used for the treatment of limb girdle muscular dystrophy (LGMD), preferably limb girdle muscular dystrophy by calpainopathy (LGMD R1).

Alternatively, the invention also relates to the use of the gene expression construct or the recombinant vector of the invention described above, for the preparation of a medicament for the treatment of limb girdle muscular dystrophy (LGMD), preferably limb girdle muscular dystrophy by calpainopathy (LGMD R1).

The gene expression construct or recombinant vector comprising said gene expression construct according to the invention are preferably used for the treatment of a mammalian patient, preferably a human, for the treatment of limb girdle muscular dystrophy (LGMD).

In a particular embodiment, LGMD is limb girdle muscular dystrophy by calpainopathy (LGMD R1).

In a particular embodiment, the gene expression construct or recombinant vector comprising said gene expression construct for use as a medicament according to the invention comprises:
- a desmin promoter consisting of the sequence of SEQ ID NO: 5 or a functional variant thereof having a polynucleotide sequence that is at least 80% identical to SEQ ID NO: 5; and eventually
- an HBB2 polyadenylation signal consisting of the sequence of SEQ ID NO: 6 or a functional variant thereof having a polynucleotide sequence that is at least 80% identical to SEQ ID NO: 6.

In a particular embodiment, the gene expression construct or recombinant vector comprising said gene expression construct for use as a medicament according to the invention comprises a transgene encoding the calpain-3 protein consisting of the sequence of SEQ ID NO: 4.

In a particular embodiment, the gene expression construct or recombinant vector comprising said gene expression construct for use as a medicament according to the invention comprises:
- a desmin promoter consisting of the sequence of SEQ ID NO: 5 or a functional variant thereof having a polynucleotide sequence that is at least 80% identical to SEQ ID NO: 5;
- a transgene encoding the calpain-3 protein consisting of the sequence SEQ ID NO: 4 or a functional variant thereof having a polynucleotide sequence that is at least 80% identical to SEQ ID NO: 4; and
- an HBB2 polyadenylation signal consisting of the sequence of SEQ ID NO: 6 or a functional variant thereof having a polynucleotide sequence that is at least 80% identical to SEQ ID NO: 6.

In a preferred embodiment, the vector for use of the invention may be an AAV9 or AAV9-rh74 vector comprising a gene expression construct as defined above comprising a transgene of interest encoding a calpain-3 protein.

The vector for use according to the invention may be administered locally with or without systemic co-delivery. In the context of the present invention, local administration denotes an administration into the muscle of the subject, such as via an intramuscular injection of the rAAV vector.

In some embodiment the vector may be administrated by a combined intramuscular and/or peripheral (such as a vascular, for example intravenous or intra-arterial, in particular intravenous) administration.

As used herein the term "intramuscular administration" refers to the administration of a vector according to the invention, or a composition comprising a vector of the invention, into the muscle preferably a skeletal muscle.

Accordingly, methods of administration of the rAAV vector include but are not limited to, intramuscular, intraperitoneal, vascular (e.g. intravenous or intra-arterial), subcutaneous, intranasal, epidural, and oral routes. In a particular embodiment, the systemic administration is a vascular injection of the rAAV vector, in particular an intravenous injection.

The amount of the vector of the invention which will be effective in the treatment of LGMD R1 can be determined by standard clinical techniques. In addition, in *vivo* and/or *in vitro* assays may optionally be employed to help predict optimal dosage ranges. The dosage of the vector of the invention administered to the subject in need thereof will vary based on several factors including, without limitation, the specific type or stage of the disease treated, the subject's age or the level of expression necessary to obtain the therapeutic effect. One skilled in the art can readily determine, based on its knowledge in this field, the dosage range required based on these factors and others. Typical doses of AAV vectors are of at least 1×10⁸ vector genomes per kilogram body weight (vg/kg), such as at least 1×10⁹ vg/kg, at least 1×10¹⁰ vg/kg, at least 1×10¹¹ vg/kg, at least 1×10¹² vg/kg at least 1×10¹³ vg/kg, at least 1×10¹⁴ vg/kg or at least 1×10¹⁵ vg/kg.

### EXAMPLES

### Example 1: Analysis of AAV integrity

**Table 1**

| Plasmid name | ITR | promoter | Tg | polyA | cassette size | % GC of Tg | nb CpG of Tg | polyA | Serotype | Titer (vg/mL) | Titer VG/ml hDesmin | Titer VG/ml PolyA HBB2 | Titer VG/ml DP | %DP |
|---|---|---|---|---|---|---|---|---|---|---|---|---|---|---|
| pAAV-hdesmine-hkozC3sfrmut-TmiR208ax2-Ampi (pG3-12566) | -gnt | Desmin | hkozC3sfrmut | SV40 | 4785 | 53% | 83 | SV40 | AAV9 | 3,20E+13 | | | | 36.4% |
| pAAV-hdesmine-hkozC3sfrmut-Ampi (pG3-12310) | gnt | Desmin | hkozC3sfrmut | SV40 | 4788 | 53% | 83 | SV40 | AAV9 | 1,10E+14 | | | | 38.1% |
| pAAV-hACTA1-hkozC3sfrmut-TmiR208ax1-Kana | -wt | acta1 | hkozC3sfrmut | HBB2 | 4350 | 53% | 83 | HBB2 | AAV-KP1 | 2,80E+10 | 8,00E+10 | 9,60E+10 | 3,00E+10 | 20,30% |
| pAAV-hACTA1-hkozC3sfrmut-TmiR208ax1-Kana | -wt | acta1 | hkozC3sfrmut | HBB2 | 4350 | 53% | 83 | HBB2 | AAVg | 5,60E+11 | 1,00E+12 | 1,10E+12 | 4,20E+11 | 24,10% |
| pAAV-Hacta1 -hkozC3sfrmut-TmiR208ax1-Kana | wt | acta1 | hkozC3sfrmut | HBB2 | 4350 | 53% | 83 | HBB2 | AAV9 | 6,70E+12 | 1,20E+13 | 1,30E+13 | 4,80E+12 | 24,00% |
| pAAV-hACTA1-hkozC3sfrmut-TmiR208ax1-Kana | wt | acta1 | hkozC3sfrmut | HBB2 | 4350 | 53% | 83 | HBB2 | AAV-KP1 | 4,70E+13 | 4,30E+13 | 5,10E+13 | 1,80E+13 | 22,80% |
| pAAV-hACTA1-hkozC3sfrmut-TmiR208ax1-Kana | -wt | acta1 | hkozC3sfrmut | HBB2 | 4350 | 53% | 83 | HBB2 | AAVg | 4,54E+13 | 4,00E+13 | 4,40E+13 | 1,60E+13 | 24,60% |
| pAAV-hACTA1-hkozC3sfrmut-TmiR208ax1-Kana | -wt | acta1 | hkozC3sfrmut | HBB2 | 4350 | 53% | 83 | HBB2 | AAV9 | 1,40E+14 | 3,80E+14 | 4,20E+14 | 1,70E+14 | 26,40% |

Series of AAV preparation containing CALPAIN transgene of SEQ ID NO: 4 have been analyzed using two digital droplet PCR (ddPCR) at each extremity of the genome: one on the promoter and the other at the polyA.

Using ddPCR allows the identification of full-length genome in droplets positive forthe two PCR. The percentage of double positive droplets is indicated in the last column of the table 1.

The results indicate that the AAV containing SGCA transgene with the ITR variant gnt (pAAV-hdesmine-hkozC3sfrmut-TmiR208ax2-Ampi (pG3-12566)) are the one with the highest percentage of double positive droplets (%DP).

This ITR variant, ITRgnt of SEQ ID NO: 1 and SEQ ID NO: 2 or SEQ ID NO: 3, is different from the ITR wt sequence by the deletion of the sequence A of the ITR. Most of the vectors with the ITR wt display a percentage of genome integrity (%DP) around 23% whereas the AAV vector with ITR variant, ITRg, are at around 36 and 38%.

## Claims

1. A gene expression construct comprising:
- an expression cassette containing a transgene encoding a calpain-3 protein;
- a modified 5'-ITR sequence of SEQ ID NO: 1; and
- a modified 3'-ITR sequence of SEQ ID NO: 2 or SEQ ID NO: 3.

2. The gene expression construct according to claim 1, wherein the transgene encoding the calpain-3 protein consists of the sequence of SEQ ID NO: 4 or a functional variant thereof having a polynucleotide sequence that is at least 80% identical to SEQ ID NO: 4.

3. The gene expression construct according to claim 1 or 2, wherein the expression cassette further comprises:
- a desmin promoter consisting of the sequence of SEQ ID NO: 5 or a functional variant thereof having a polynucleotide sequence that is at least 80% identical to SEQ ID NO: 5; and optionally
- an HBB2 polyadenylation signal consisting of the sequence of SEQ ID NO: 6 or a functional variant thereof having a polynucleotide sequence that is at least 80% identical to SEQ ID NO: 6.

4. The gene expression construct according to claim 3, wherein the expression cassette comprises:
- a desmin promoter consisting of the sequence of SEQ ID NO: 5 or a functional variant thereof having a polynucleotide sequence that is at least 80% identical to SEQ ID NO: 5;
- a transgene encoding the calpain-3 protein consisting of the sequence of SEQ ID NO: 4 or a functional variant thereof having a polynucleotide sequence that is at least 80% identical to SEQ ID NO: 4; and
- an HBB2 polyadenylation signal consisting of the sequence of SEQ ID NO: 6 or a functional variant thereof having a polynucleotide sequence that is at least 80% identical to SEQ ID NO: 6.

5. A recombinant vector comprising the gene expression construct according to any one of claims 1 to 4.

6. The recombinant vector according to claim 5, which isa viral vector.

7. The recombinant vector according to claim 6, wherein the viral vector is a recombinant adeno-associated virus (rAAV) vector.

8. The recombinant vector according to claim 7, wherein said rAAV vector has an AAV9 or AAV9rh74 capsid.

9. The gene expression construct according to any one of claims 1 to 4, or the recombinant vector according to any one of claims 5 to 8, for use as a medicament.

10. The gene expression construct or the recombinant vector for use according to claim 9, for the treatment of limb girdle muscular dystrophy by calpainopathy.
